# EUROPEAN PATENT APPLICATION

(11) **EP 2 317 361 A1**
(43) Date of publication of application: **04.05.2011**
(21) Application number: 09382230.2
(22) Date of filing: 27.10.2009
(51) Int. Cl.: G02B 21/00

(54) **Capilaroscopy device**

(71) Applicant: Besoli Minguela, Gloria, 08018 Barcelona (ES)
(72) Inventor: Besoli Minguela, Gloria, 08018 Barcelona (ES)
(74) Representative: Ponti Sales, Adelaida

(57) **Abstract**

The examination device for capillaroscopy of the present invention comprises a microscope (1) and a light that lights a zone of the body (2) to examine said zone, and it is characterised in that the device also comprises a support (3) for said zone of the body to be explored, said support (3) being integral with said microscope (1).

Said support (3) has an ergonomic shape according to the zone of the body (2) to be examined.

It permits that any movement of the zone of the body to examine is transmitted to said microscope through the support, so that the quality of the image obtained of the zone to be examined is not affected by these movements.

## Description

The present invention refers to an examination device for medical diagnostic, particularly for capillaroscopy, comprising a microscope and a light to light the zone to be examined, e.g. the blood capillaries of a part of the body.

### BACKGROUND OF THE INVENTION

The capillaroscopy is a diagnostic medical technique, which uses a increasing lens or a stereoscopic microscope with a cold light source, which permits to see the capillaries to be examined.

If wished, it is possible to use liquid substances to enhance the transparency of the zone to be examined.

The capillaroscopy devices currently known comprise a base plate-like support, on which the hand of the patient rests, so that the tip of the finger to be examined remains placed under the microscope lighted by the light source.

The problem of these known capillaroscopy devices is that if the finger or hand moves during the examination, the quality of the obtained image decreases, making the diagnostic difficult. This fact is increased if the patient has any disease related with the fingers, e.g. if he/she cannot extend completely the fingers, which involves severe pain for the user for a correct examination.

In the practice, this drawback means that it is necessary to repeat the examination all the times needed to obtain the correct diagnostic.

Therefore, it is evident the need of an examination device that can be used in capillaroscopy that minimizes or removes these drawbacks, so that the image to be obtained from the examination is always with a quality enough to permit the diagnostic.

### SUMMARY OF THE INVENTION

With the examination device of the invention said drawbacks can be solved, presenting other advantages that will be described.

The examination device for capillaroscopy of the present invention comprises a microscopy and a light that lights a zone of the body to examined said zone, and it is characterised in that it also comprises a support for said zone of the body to be examined, said support being integral with said microscopy.

Thanks to this feature, any movement of the zone of the body to be examined is transmitted to said microscopy through the support, so that the quality of the obtained image of the zone to examine is not affected by these movements.

Advantageously, said support has an ergonomic shape according to the zone of the body to examine.

According to a preferred embodiment, to analyse the nails of the fingers, said support has a spherical or semi-spherical shape. This support shape permits the patients with difficulties to extend the fingers of the hand not to do it to obtain a quality image of the capillaries of the nails of the fingers.

Preferably, said support is linked with said microscope by an arm, said arm being adjustable to adjust the position of the microscope with respect to the support.

### BRIEF DESCRIPTION OF THE DRAWINGS

For a better understanding of that disclosed some drawings are attached in which, diagrammatically and only a non-limitative example, a practical case of embodiment is shown.

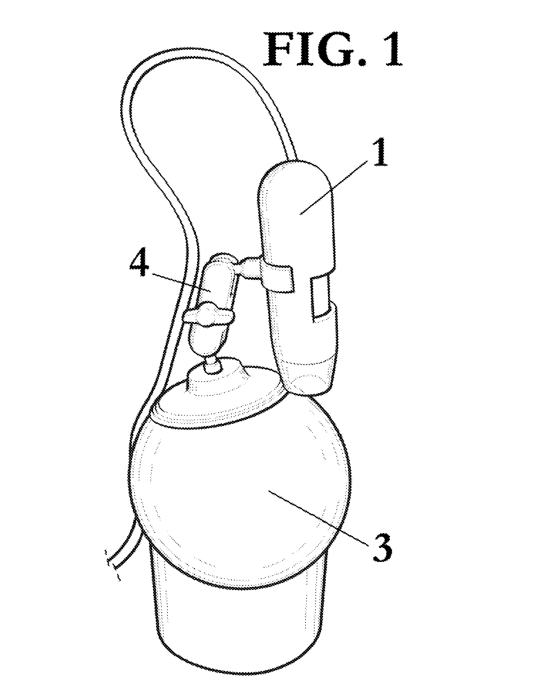

Fig. 1 is a perspective view of the examination device for capillaroscopy of the present invention; and
Fig. 2 is a perspective view of the examination device for capillaroscopy of the present invention during its use with the hand of a patient.

### DESCRIPTION OF A PREFERRED EMBODIMENT

Firstly, it must be pointed out that the examination device of the present invention is specially designed to be used in capillaroscopy, however, it could be also used to examine any part of the body of a patient, changing the support for the specific part of the body.

As it can be seen in the figures, the examination device of the present invention comprises a microscope 1 provided with a light, said light lighting the zone of the body to be explored, e.g. the capillaries of the nail fold of a finger of the hand of the patient 2.

The image obtained by the microscope 1 is sent to a computer for processing the image (not shown) by suitable means. In the screen of the computer said image can be analysed for the corresponding diagnostic.

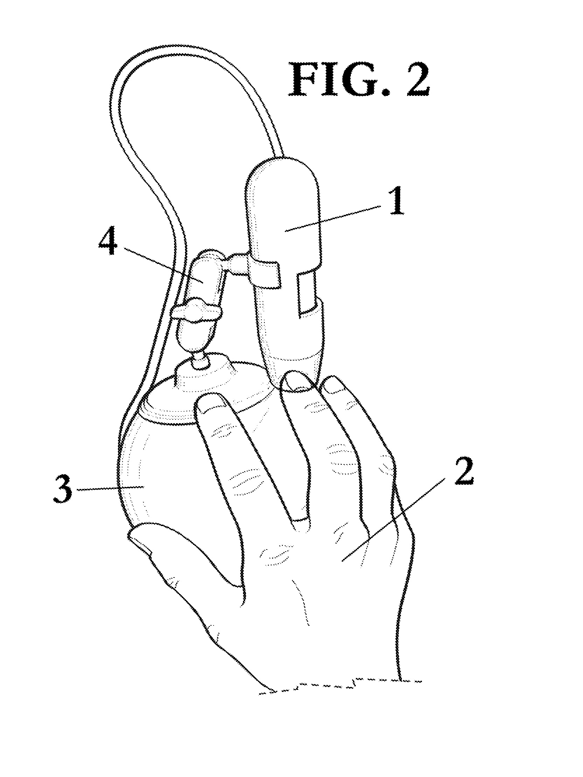

According to the invention, the examination device of the present invention also comprises a support 3 integral with said microscope 1. This support 3 is provided for the zone of the body to be explored, in the case of the embodiment shown in Fig. 2 the finger of a hand 2, that rest against the support.

Therefore, any movement, even little, of the zone of the body to be explored will make the microscope also to be moved correspondingly, so that the quality of the image will not be affected substantially by said movement.

According to the embodiment shown in the figures, said support 3 is ergonomic, particularly a sphere shape, if it is used for supporting the hand 2 of a patient. This spherical shape of the support 3 permits the fingers of the patient not to be completely extended for a correct examination of them, thanks to the curvature of said support 3.

Obviously, said support 3 can be of any suitable shape, e.g. semi-spherical or that mimics the complementary anatomic shape of the zone to be explored.

The connection between the support 3 and the microscope 1 is done preferably by a swinging arm 4, through which the position of the microscope 1 with respect to the support 3 can be adjusted. However, it must be pointed out that the connection between the support 3 and the microscope 1 can be done in any suitable way, if any movement of the support 3 is transmitted to the microscope 1 and the position of said microscope 1 with respect to the support 3 can be adjusted easily.

Even though reference is made to a specific embodiment of the invention, it is apparent for a person skilled in the art that the examination device described is susceptible of numerous variations and modifications, and that all the details cited can be substituted by other technically equivalent ones, without departing from the scope of protection defined by the attached claims.

## Claims

1. Examination device for capillaroscopy, comprising a microscope (1) and a light that lights a zone of the body (2) to examine said zone, **characterised in that** the device also comprises a support (3) for said zone of the body to be explored, said support (3) being integral with said microscope (1).

2. Examination device according to claim 1, wherein said support (3) has an ergonomic shape according to the zone of the body (2) to be examined.

3. Examination device according to claim 1 or 2, wherein said support (3) has a spherical or semi-spherical shape.

4. Examination device according to any of the previous claims, wherein said support (3) is linked with said microscope (1) by an arm (4).

5. Examination device according to claim 4, wherein said arm (4) is adjustable to adjust the position of the microscope (1) with respect to the support (3).
